Europäisches Patentamt

European Patent Office

Office européen des brevets

(19)

(11) Numéro de publication : **0 409 716 B1**

(12) **FASCICULE DE BREVET EUROPEEN**

(45) Date de publication du fascicule du brevet :
**15.12.93 Bulletin 93/50**

(51) Int. Cl.$^5$ : **C07C 209/34,** C07C 211/52

(21) Numéro de dépôt : **90402048.4**

(22) Date de dépôt : **17.07.90**

(54) **Procédé d'hydrogénation de dérivés halogéno nitroaromatiques en présence d'iodures.**

(30) Priorité : **20.07.89 FR 8909765**

(43) Date de publication de la demande :
**23.01.91 Bulletin 91/04**

(45) Mention de la délivrance du brevet :
**15.12.93 Bulletin 93/50**

(84) Etats contractants désignés :
**AT BE CH DE DK ES FR GB GR IT LI LU NL SE**

(56) Documents cités :
**FR-A- 2 245 615**
**GB-A- 1 498 722**

(73) Titulaire : **RHONE-POULENC CHIMIE**
**25, quai Paul Doumer**
**F-92408 Courbevoie Cédex (FR)**

(72) Inventeur : **Grosselin, Jean-Michel**
**12, allée des Erables**
**F-69340 Francheville (FR)**
Inventeur : **Cordier, Georges**
**50, Chemin des Hermières**
**F-69340 Francheville (FR)**

(74) Mandataire : **Ricalens, François et al**
**RHONE-POULENC CHIMIE Service Brevets**
**Chimie 25, quai Paul Doumer**
**F-92408 Courbevoie (FR)**

**Description**

La présente invention concerne un procédé d'hydrogénation de composés nitrés et halogénés aromatiques. Elle concerne plus particulièrement la préparation d'amines halogénées.

Lors de la mise en oeuvre d'un procédé d'hydrogénation sur un dérivé aromatique nitré portant des atomes d'halogène liés au noyau aromatique, il se produit en même temps que la transformation du groupe nitro en groupe aminé un phénomène de déshalogénation avec perte des atomes d'halogène sous forme d'hydrohalogènures.

Ce phénomène est connu depuis très longtemps puisqu'il a été décrit en 1904 par P. Sabatier et A. Mailhe.

De nombreux brevets ont été déposés pour éviter cette réaction secondaire tout en permettant de conserver une activité correcte au catalyseur.

Ces brevets peuvent être divisés en deux groupes : ceux utilisant comme catalyseur d'hydrogénation le nickel de Raney et ceux utilisant le platine ou le palladium.

Tous ces brevets décrivent l'utilisation d'un catalyseur modifié.

Dans le premier groupe de brevets décrivant l'utilisation du nickel de Raney on peut citer les brevets U.S 3067253, G.B 1191610, J 73-49728, G.B 1498722 et FR 2245615.

Le brevet U.S 3067253 décrit l'utilisation de nickel de Raney auquel est adjoint un hydroxyde de calcium ou de magnésium. Les températures réactionnelles sont néanmoins toujours basses (25 à 60°C) afin d'éviter la déshalogénation, ce qui ne permet pas de pouvoir utiliser ces procédés industriellement.

Le brevet G.B 1191610 décrit l'utilisation du nickel de Raney associé à la présence d'un thiocyanate. Ce procédé ne permet qu'une hydrogénation lente (4 heures à 8 heures) et le catalyseur est altéré lors de l'hydrogénation, ce qui ne permet pas d'envisager un procédé en continu.

Le brevet J 73-49728 décrit l'utilisation du nickel de Raney associé à la présence d'une alkylamine, d'une alkanolamine ou d'une base hétérocyclique. Dans ce brevet la température d'hydrogénation est limitée comme dans le brevet U.S précédemment mentionné à 60°C.

Cette température ne permet pas une exploitation industrielle satisfaisante car la productivité du procédé à ces températures est insuffisante. Il est même indiqué dans le brevet FR 2245615 que le procédé du brevet J 73-49728 ne permet pas d'éviter la déhalogénation de façon satisfaisante puisque au moins 5% de l'aniline obtenue est déshalogénée.

Le brevet G.B 1498722 décrit l'utilisation de nickel de Raney avec un trialkylphosphite. La température de réaction est d'environ 100°C mais le taux de déshalogénation est très élevé puisqu'il varie entre 2 et 8%. Ce procédé n'est donc pas envisageable industriellement.

Le dernier brevet décrivant l'utilisation du nickel de Raney est le brevet FR 2245615 qui l'associe avec un inhibiteur de déshalogénation choisi parmi le dicyandiamide, le cyamide et le cyanamide calcique. La température de la réaction d'hydrogénation est comprise entre 50 et 130°C et les taux de déshalogénation sont toujours inférieurs à 0,15%.

Dans le deuxième groupe de brevets décrivant l'utilisation de métaux de la mine du platine on peut citer les brevets FR 2330669 et FR 2127092.

Le brevet FR 2330669 décrit l'utilisation comme catalyseur d'hydrogénation de composés nitroaromatiques chlorés, du platine déposé sur charbon et inhibé par la présence d'un dérivé soufré choisi parmi les thioéthers et les disulfures. Le taux de déshalogénation est très faible (0,01 à 0,08%), ce faible taux de déshalogénation provient en grande partie du platine qui même en l'absence de dérivé soufré, ne provoque pas de déshalogénation

Le brevet FR 2127092 décrit la préparation d'un catalyseur au platine déposé sur charbon qui est sulfuré. La préparation de ce catalyseur consiste à procéder d'abord à une hydrogénation du catalyseur puis à sulfurer ensuite celui-ci par addition d'hydrogène sulfuré selon une quantité variant entre 0,45 et 0,55 mole d'hydrogène sulfuré par mole d'hydrogène absorbée.

D'une part la préparation du catalyseur est difficile, d'autre part l'utilisation d'un catalyseur déposé sur charbon ne permet pas une décantation facile de la masse catalytique et rend ainsi très difficile l'exploitation du procédé selon un mode continu. L'utilisation de platine, catalyseur très onéreux a fait reculer l'industrie quant à la mise en oeuvre industrielle d'un tel procédé.

La présente invention a su à partir du nickel, catalyseur d'hydrogénation le moins onéreux et aisément décantable donc particulièrement bien adapté pour l'hydrogénation continue , l'associer avec un inhibiteur de déshalogénation de façon à lui apporter toutes les qualités requises pour l'hydrogénation des dérivés halogéno nitroaromatiques.

Elle consiste à mettre en présence le dérivé halogéno nitroaromatique ou polyhalogéno nitroaromatique avec un catalyseur d'hydrogénation constitué de nickel de Raney en présence d'iodure. L'iodure est choisi parmi les iodures alcalins ou d'ammonium. On préfère utiliser l'iodure de potassium.

Les dérivés halogéno nitroaromatique qui peuvent être hydrogénés selon le procédé de la présente invention répondent à la formule suivante :

$$(Z)_q \, (Y)_p \, (X)_n - Ar - NO_2$$

dans laquelle

- Ar représente un radical mono, polycyclique ou hétérocyclique éventuellement substitué par un groupe alkyle contenant 1 à 4 atomes de carbone
- X,Y,Z représentent chacun un halogène choisi parmi le fluor, le chlore et le brome
- n,p,q représentent chacun un nombre compris entre 0 et 5, la somme n+p+q étant égale ou supérieure à 1.

On préfère utiliser un composé halogéno nitroaromatique monocyclique contenant un à trois atomes d'halogène choisi parmi le chlore et le fluor fixés sur le noyau et tout particulièrement l'invention s'applique aux dérivés suivants :

les chloronitrobenzènes

les fluoronitrobenzènes

les dichloronitrobenzènes

les monochloro monofluoro nitrobenzènes

les trichloronitrobenzènes

les chloronitrométhylbenzènes

les fluoronitrométhylbenzènes

On effectue l'hydrogénation des dérivés halogéno-nitroaromatiques à l'aide du nickel de Raney et de l'iodure dans les conditions usuelles d'hydrogénation. En raison du fort potentiel de non déshalogénation qu'apportent les iodures il est tout à fait possible de réaliser l'hydrogénation à une température comprise entre 70 et 150°C.

Elle est réalisée préférentiellement entre 70 et 100°C ce qui permet d'obtenir des productivités (quantité d'amine formée par heure et par volume de milieu réactionnel)

comparatives avec les productivités des procédés visant la préparation d'amines non halogénées.

Un autre avantage du catalyseur selon l'invention que n'apportaient pas les catalyseurs déposés sur charbon est la facilité de sa mise en oeuvre par des procédés réalisés en continu. En effet, la séparation du nickel de Raney est nettement plus facile que celle des catalyseurs déposés sur charbon.

Un dernier avantage du catalyseur selon l'invention est la facilité de sa mise en oeuvre, aucune préparation préalable du catalyseur n'est nécessaire comme dans le brevet FR 2127092 qui nécessite une hydrogénation du catalyseur suivi de sa sulfuration, dans le présent procédé il est seulement nécessaire d'introduire tous les réactifs dans le réacteur d'hydrogénation ; le nickel de Raney, le dérivé halogénonitroaromatique, l'iodure, le solvant puis de pressuriser le réacteur avec de l'hydrogène.

Le procédé de l'invention peut être mis en oeuvre en l'absence de solvant ou dans tout solvant inerte dans les conditions de la réaction tel que par exemple :

l'eau

les alcools tels que le méthanol, l'éthanol, l'isopropanol

les solvants aromatiques tels que le toluène, le xylène.

On préfère utiliser le méthanol.

Pour une meilleure mise en oeuvre de l'invention on préfère utiliser une quantité pondérale de nickel de Raney par litre de milieu réactionnel comprise entre 1 et 150 g et de préférence comprise entre 1 et 5 g et une quantité molaire d'iodure calculée par litre de milieu réactionnel comprise entre $10^{-5}$ mole et $10^{-3}$ mole.

Le procédé pouvant être mis en oeuvre en continu, la quantité de dérivé halogéno nitroaromatique ne peut être établie de manière statique mais sous forme de flux. Ainsi, des débits d'environ 1 à 3 moles par litre de milieu réactionnel et par heure sont tout à fait recommandés.

La pression d'hydrogène est avantageusement comprise entre 1 et 100 bars et de préférence entre 1 et 40 bars et encore plus préférentiellement entre 5 et 25 bars.

La présente invention sera plus complètement décrite à l'aide (des exemples suivants qui ne doivent en aucun cas être considérés comme limitatifs de l'invention.

Exemple 1

Dans un autoclave de 125 ml en acier inox, on charge 0,12 g de nickel de Raney, x moles de KI et 40 ml de méthanol à 95%. Après fermeture, le réacteur est purgé plusieurs fois avec de l'azote puis de l'hydrogène.

La pression est fixée à 20 bars. Le mélange réactionnel est chauffé à la température de 100°C. Dès que celle-ci est atteinte, on procède à l'injection du substrat (10 g d'un mélange para-fluoronitrobenzène/ortho-fluoronitrobenzène de composition molaire 90%/10%).

En fin de réaction, le réacteur est refroidi à température ambiante puis dégazé.

La masse réactionnelle est filtrée sur fritté et le filtrat est analysé en CPG.

Le taux de transformation atteint 100%

| I | % Aniline | ACTIVITE (mole $H_2$/g Ni x h) |
|---|---|---|
| 0 | 2,2 | 1,50 |
| $10^{-5}$M | 1,3 | 1,22 |
| $10^{-4}$M | 0,6 | 1,05 |
| $10^{-3}$M | 0,1 | 0,95 |
| $10^{-2}$M | 0 | 0 |

## Exemple 2

On reproduit l'exemple 1 en utilisant une concentration molaire de KI dans le milieu réactionnel de 10-3 M. Le catalyseur après décantation est recyclé 5 fois.

| Recyclage | % Aniline | ACTIVITE (mole $H_2$/gNixh) |
|---|---|---|
| 1 | 0,05 | 0,85 |
| 2 | 0,1 | 0,95 |
| 3 | 0,2 | 0,90 |
| 4 | 0,2 | 0,94 |
| 5 | 0,2 | 0,87 |
| 6 | 0,2 | 0,88 |

## Revendications

1. Procédé de préparation des dérivés halogéno- -aminoaromatiques caractérisé en ce que l'on met présence un dérivé halogénonitroaromatique avec du nickel de Raney et de l'hydrogène en présence d'un iodure.

2. Procédé selon la revendication 1, caractérisé en ce que le dérivé halogénonitroaromatique répond à la formule (I).

$$(Z)_q (Y)_p (X)_n - Ar - NO_2$$

dans laquelle
   - Ar représente un dérivé aromatique mono, poly ou hétérocyclique éventuellement substitué par un groupe alkyle contenant 1 à 4 atomes de carbone
   - X,Y,Z représentent un halogène choisi parmi le fluor, le chlore et le brome
   - n,p,q, représentent chacun un nombre entier compris entre 0 et 5, la somme n+p+q étant supérieure ou égale à 1.

3. Procédé selon la revendication 2, caractérisé en ce que Ar représente un radical aromatique monocyclique et X et Y représentent le chlore et/ou le fluor, q est 0 et la somme n+p est supérieure ou égale à 1 et inférieure ou égale à 3.

4. Procédé selon la revendication 1, caractérisé en ce que l'iodure est choisi parmi les iodures alcalins ou d'ammonium.

5. Procédé selon la revendication 4, caractérisé en ce que l'iodure est l'iodure de potassium.

6. Procédé selon la revendication 1, caractérisé en ce que la réaction est réalisée en l'absence de solvant ou dans un solvant choisi parmi les alcools, les dérivés aromatiques.

7. Procédé selon la revendication 7, caractérisé en ce que le solvant est le méthanol.

8. Procédé selon la revendication 1, caractérisé en ce que la quantité de nickel utilisée est comprise entre 1 et 150 g par litre de milieu réactionnel et de préférence entre 1 et 5 g par litre.

9. Procédé selon la revendication 1, caractérisé en ce que la quantité molaire d'iodure calculée par litre de milieu réactionnel est comprise entre $10^{-3}$M et $10^{-5}$M.

10. Procédé selon la revendication 1, caractérisé en ce que la température de la réaction est comprise entre 70°C et 150°C et de préférence entre 70°C et 100°C.

## Patentansprüche

1. Verfahren zur Herstellung von halogenierten aromatischen Aminoverbindungen, dadurch gekennzeichnet, daß man eine halogenierte nitroaromatische Verbindung mit Raney-Nickel und Wasserstoff in Gegenwart eines Iodids zusammenbringt.

2. Verfahren nach Anspruch 1, dadurch gekennzeichnet, daß die halogenierte nitroaromatische Verbindung der Formel (I)

$$(Z)_q(Y)_p(X)_n - Ar - NO_2$$

entspricht, in der
   - Ar den Rest einer aromatischen monocyclischen, polycyclischen oder heterocyclischen Verbindung, der gegebenenfalls durch eine Alkylgruppe mit 1 bis 4 Kohlenstoffatomen substituiert ist, darstellt,
   - X, Y und Z jeweils ein Halogen(atom), ausgewählt aus Fluor, Chlor und Brom, bedeuten und
   - n, p, q jeweils eine ganze Zahl im Bereich von 0 bis 5 sind, wobei die Summe n+p+q größer oder gleich 1 ist.

3. Verfahren nach Anspruch 2, dadurch gekennzeichnet, daß Ar für eine monocyclische aromatische Gruppe steht, X und Y für Chlor und/oder Fluor stehen, q 0 ist und die Summe n+p größer oder gleich 1 und kleiner oder gleich 3 ist.

4. Verfahren nach Anspruch 1, dadurch gekennzeichnet, daß das Iodid aus den Alkali- oder Ammoniumiodiden ausgewählt wird.

5. Verfahren nach Anspruch 4, dadurch gekennzeichnet, daß das Iodid Kaliumiodid ist.

6. Verfahren nach Anspruch 1, dadurch gekennzeichnet, daß die Reaktion in Abwesenheit eines Lösungsmittels oder in einem Lösungsmittel, ausgewählt aus Alkoholen und aromatischen Verbindungen, durchgeführt wird.

7. Verfahren nach Anspruch 7, dadurch gekennzeichnet, daß das Lösungsmittel Methanol ist.

8. Verfahren nach Anspruch 1, dadurch gekennzeichnet, daß die eingesetzte Nickelmenge 1 bis 150 g je Liter Reaktionsmedium, vorzugsweise 1 bis 5 g je Liter beträgt.

9. Verfahren nach Anspruch 1, dadurch gekennzeichnet, daß die berechnete molare Menge Iodid je Liter im Bereich von $10^{-3}$ M bis $10^{-5}$ M liegt.

10. Verfahren nach Anspruch 1, dadurch gekennzeichnet, daß die Reaktionstemperatur im Bereich von 70°C bis 150°C, vorzugsweise im Bereich von 70°C bis 100°C liegt.

## Claims

1. A process for preparing haloaminoaromatic compounds characterised in that a halonitroaromatic compound is brought into contact with Raney nickel and hydrogen in the presence of an iodide.

2. A process according to Claim 1 in which the halo nitroaromatic compound corresponds to the formula:

$$(Z)_q (Y)_p (X)_n - Ar - NO_2$$

in which

Ar represents a monocyciic, polycylic or heterocyclic radical, optionally having a $C_{1-4}$ alkyl substituent;

each of X, Y and Z represents a halogen selected from fluorine, chlorine and bromine;

and each of n, p and q represents an integer from 0 to 5, the sum n+p+q being equal to or greater than 1.

3. A process according to Claim 2 characterised in that Ar represents a monocyclic aromatic radical and X and Y represent chlorine and/or fluorine, q is 0 and the sum n+p is from 1 to 3 inclusive.

4. A process according to Claim 1 characterised in that the iodide is chosen from alkali metal iodides and ammonium iodide.

5. A process according to Claim 4 characterised in that the iodide is potassium iodide.

6. A process according to Claim 1 characterised in that the reaction is carried out in the absence of solvent or in a solvent chosen from alcohols and aromatic derivatives.

7. A process according to Claim 6 characterised in that the solvent is methanol.

8. A process according to Claim 1 characterisea in that the quantity of nickel used is from 1 to 150 g per litre of reaction medium, preferably from 1 to 5 g per litre.

9. A process according to Claim 1 characterised in that the molar quantity of iodide calculated per litre of the reaction medium is from $10^{-3}M$ to $10^{-5}M$.

10. A process according to Claim 1 characterised in that the reaction temperature is from 70 to 150°C, preferably from 70 to 100°C.